# EUROPEAN PATENT SPECIFICATION

(11) **EP 4 017 596 B1**
(45) Date of publication and mention of the grant of the patent: **11.11.2026**
(21) Application number: 21714764.4
(22) Date of filing: 09.03.2021
(51) Int. Cl.: A61Q 5/00, A61Q 5/02, A61Q 15/00, A61Q 19/00, A61Q 19/10, A61K 8/36, A61K 8/365

(54) **PERSONAL CARE COMPOSITIONS**
KÖRPERPFLEGEZUSAMMENSETZUNGEN
COMPOSITIONS DE SOINS PERSONNELS

(30) Priority: 09.03.2020 US 202062987049 P; 09.03.2020 US 202062987023 P
(43) Date of publication of application: 29.06.2022
(73) Proprietor: Colgate-Palmolive Company, New York, NY 10022 (US)
(72) Inventor: BHARDWAJ, Vinay, Piscataway, New Jersey 08854 (US); FAN, Aixing, New Jersey 08807 (US); NESTA, Jason, Somerset, New Jersey 08873 (US); BOYD, Thomas, Metuchen, New Jersey 08840 (US); LI, Min, Bridgewater, New Jersey 08807 (US)
(74) Representative: Wichmann, Hendrik
(86) International application number: PCT/US2021/021426
(87) International publication number: WO 2021/183462

(56) References cited:
- WO-A1-2018/022016
- WO-A1-2020/185654
- JP-A- 2004 089 177
- US-A- 5 785 962
- US-A1- 2011 256 082
- DATABASE GNPD [online] MINTEL; 27 November 2012 (2012-11-27), ANONYMOUS: "Super-Mud Clearing Treatment", XP055814782, retrieved from https://www.gnpd.com/sinatra/recordpage/1921209/ Database accession no. 1921209
- DATABASE GNPD [online] MINTEL; 10 November 2017 (2017-11-10), ANONYMOUS: "Promise Keeper Blemish Facial", XP055814783, retrieved from https://www.gnpd.com/sinatra/recordpage/5196555/ Database accession no. 5196555
- DATABASE GNPD [online] MINTEL; 2 April 2019 (2019-04-02), ANONYMOUS: "Supertoner Exfoliating Acid Solution", XP055814786, retrieved from https://www.gnpd.com/sinatra/recordpage/6457009/ Database accession no. 6457009
- DATABASE GNPD [online] MINTEL; 29 April 2019 (2019-04-29), ANONYMOUS: "Superserum 6-Acid Refining Treatment", XP055814788, retrieved from https://www.gnpd.com/sinatra/recordpage/6521601/ Database accession no. 6521601
- DATABASE GNPD [online] MINTEL; 11 June 2014 (2014-06-11), ANONYMOUS: "Clinical Concentrate Radiance Booster", XP055814790, retrieved from https://www.gnpd.com/sinatra/recordpage/2350479/ Database accession no. 2350479
- DATABASE GNPD [online] MINTEL; 29 September 2011 (2011-09-29), ANONYMOUS: "Milk Body Lotion", XP055814794, retrieved from https://www.gnpd.com/sinatra/recordpage/1632704/ Database accession no. 1632704
- DATABASE GNPD [online] MINTEL; 4 November 2011 (2011-11-04), ANONYMOUS: "E.V.E. Essential Vital Elements Serum Source", XP055692005, retrieved from https://www.gnpd.com/sinatra/recordpage/1666406/ Database accession no. 1666406
- DATABASE GNPD [online] MINTEL; 10 October 2006 (2006-10-10), ANONYMOUS: "Micro Exfoliating Oxygenating Gel", XP055814797, retrieved from https://www.gnpd.com/sinatra/recordpage/598917/ Database accession no. 598917
- DATABASE GNPD [online] MINTEL; 22 September 2014 (2014-09-22), ANONYMOUS: "Intimate Wash", XP055814801, retrieved from https://www.gnpd.com/sinatra/recordpage/2677521/ Database accession no. 2677521
- DATABASE GNPD [online] MINTEL; 10 April 2017 (2017-04-10), ANONYMOUS: "Acne On The Spot Serum", XP055814802, retrieved from https://www.gnpd.com/sinatra/recordpage/4688909/ Database accession no. 4688909
- DATABASE GNPD [online] MINTEL; 24 June 2005 (2005-06-24), ANONYMOUS: "Intensity Classic Deo Roll-On", XP055814826, retrieved from https://www.gnpd.com/sinatra/recordpage/371579/ Database accession no. 371579
- DATABASE GNPD [online] MINTEL; 10 April 2018 (2018-04-10), ANONYMOUS: "Shaveless Hair Minimizing Anti-Perspirant Deodorant Roll-On", XP055814852, retrieved from https://www.gnpd.com/sinatra/recordpage/5574907/ Database accession no. 5574907

## Description

### BACKGROUND

Probiotics are well known to have beneficial effects on skin health. Much effort has been put forth to improve the skin microflora balance. In particular, it is desirable to inhibit harmful bacteria, while promoting the growth of beneficial bacteria. However, the ability to provide this dual benefit still remains a challenge.

In addition, skin tends to lose its elasticity and/or firmness as it ages. Current options for maintaining skin elasticity and firmness are sub-optimal.

As such, embodiments of the present invention are designed to provide these, and other, benefits. Mintel database entry "Super-Mud Clearing Treatment" (XP055814782, Database accession no. 1921209) discloses a personal care composition to target pores, problem skin, blemishes, pimples, spots, black and white heads, razor bumps and in-grown hair and comprising in a cosmetically acceptable carrier, lactic acid, mandelic acid, pyruvic acid and glycolic acid. Mintel database entry "Promise Keeper Blemish Facial" (XP055814783, Database accession no. 5196555) discloses a personal care composition to repair, regenerate and re-train skin and comprising in a cosmetically acceptable carrier, lactic acid, mandelic acid and pyruvic acid. Mintel database entry "Supertoner Exfoliating Acid Solution" (XP055814786, Database accession no. 6457009) discloses a personal care composition for providing refreshed, toned, refined skin and for removing dead surface cells on the skin, comprising in a cosmetically acceptable carrier, lactic acid, mandelic acid, pyruvic acid, tartaric acid, glycolic acid and citric acid. Mintel database entry "Superserum 6-Acid Refining Treatment" (XP055814788, Database accession no. 6521601) discloses a personal care composition used as a skin-plumping and exfoliating serum, comprising in a cosmetically acceptable carrier, lactic acid, mandelic acid, pyruvic acid, glycolic acid, tartaric acid and sorbic acid. Mintel database entry "Clinical Concentrate Radiance Booster" (XP055814790, Database accession no. 2350479) discloses a personal care composition to transform dull skin into a youthful-looking, glowing, luminous complexion, comprising in a cosmetically acceptable carrier, lactic acid, mandelic acid, pyruvic acid, glycolic acid and tartaric acid.

### BRIEF SUMMARY

The present invention provides personal care compositions comprising: a complex comprising a plurality of short chain fatty acids; and a cosmetically acceptable carrier as further defined in the claims.

The compositions can be used in methods of treating a body surface of a mammal (e.g. skin) in need thereof, the method comprising administering to the body surface of the patient, an effective amount of a personal care composition described herein or a SCFA Blend described herein.

### BRIEF DESCRIPTION OF THE DRAWINGS

Figure 1 depicts a comparison of the anti-tyrosinase activity provided by an exemplary composition of the present invention and individual short chain fatty acids.
Figure 2 depicts a comparison of the anti-collagenase activity provided by another exemplary composition of the present invention and individual short chain fatty acids.
Figure 3 depicts a comparison of the anti-tyrosinase activity provided by a third exemplary composition of the present invention and individual short chain fatty acids.
Figure 4 depicts a comparison of the anti-collagenase activity provided by a fourth exemplary composition of the present invention and individual short chain fatty acids.

### DETAILED DESCRIPTION

For illustrative purposes, the principles of the present invention are described by referencing various exemplary embodiments thereof. Although certain embodiments of the invention are specifically described herein, one of ordinary skill in the art will readily recognize that the same principles are equally applicable to, and can be employed in other apparatuses and methods. Before explaining the disclosed embodiments of the present invention in detail, it is to be understood that the invention is not limited in its application to the details of any particular embodiment shown. The terminology used herein is for the purpose of description and not of limitation.

As used herein and in the appended claims, the singular forms "a", "an", and "the" include plural references unless the context dictates otherwise. The singular form of any class of the ingredients refers not only to one chemical species within that class, but also to a mixture of those chemical species. The terms "a" (or "an"), "one or more" and "at least one" may be used interchangeably herein. The terms "comprising", "including", and "having" may be used interchangeably. The term "include" should be interpreted as "include, but are not limited to". The term "including" should be interpreted as "including, but are not limited to".

As used throughout, ranges are used as shorthand for describing each and every value that is within the range.

The term "about" when referring to a number means any number within a range of 10% of the number. For example, the phrase "about 2.0 wt%" refers to a number between and including 1.800 wt% and 2.200 wt%.

The abbreviations and symbols as used herein, unless indicated otherwise, take their ordinary meaning. The abbreviation "wt%" means percent by weight with respect to the personal care composition. The symbol "°" refers to a degree, such as a temperature degree or a degree of an angle. The symbols "h", "min", "mL"," nm", "µm" means hour, minute, milliliter, nanometer, and micrometer, respectively. The abbreviation "UV-VIS" as referring to a spectrometer or spectroscopy, means Ultraviolet-Visible. The abbreviation "rpm" means revolutions per minute.

The phrase "MRS agar" refers to De Man, Rogosa and Sharpe agar, which is a selective culture medium designed to favor the growth of *Lactobacillus.* The phrase "TSB medium" refers to tryptic soy broth or trypticase soy broth, which is used in microbiology laboratories as a culture broth to grow aerobic bacteria. The phrase "PBS wash" refers to phosphate-buffered saline wash. The abbreviation "qPCR" is quantitative polymerase chain reaction.

When referring to chemical structures, and names, the symbols "C", "H", and "O" mean carbon, hydrogen, and oxygen, respectively. The symbols "-", "=" and "≡" mean single bond, double bond, and triple bond respectively.

Any member in a list of species that are used to exemplify or define a genus, may be mutually different from, or overlapping with, or a subset of, or equivalent to, or nearly the same as, or identical to, any other member of the list of species. Further, unless explicitly stated, such as when reciting a Markush group, the list of species that define or exemplify the genus is open, and it is given that other species may exist that define or exemplify the genus just as well as, or better than, any other species listed.

For readability purposes, the chemical functional groups are in their adjective form; for each of the adjective, the word "group" is assumed. For example, the adjective "alkyl" without a nouns thereafter, should be read as "an alkyl group".

A systematic search for a probiotic composition that has beneficial effects on a person's skin has been undertaken. The search has yielded a unique probiotics complex which is the fermentation product of *Lactobacillus rhamnosus* with 2.5% xylitol. In vitro testing has shown that this technology balanced skin microflora by selectively inhibiting the growth of undesirable skin bacteria and maintaining/promoting the growth of desirable bacteria. However, the composition of the biological complex is highly complicated and largely unknown raising potential safety and regulatory concerns.

*Escherichia coli* is a Gram-negative, facultative anaerobic, rod-shaped, coliform bacterium of the genus Escherichia that is commonly found in the lower intestine of warm-blooded organisms (endotherms). Most E. coli strains are harmless, but some serotypes can cause serious food poisoning in their hosts, and are occasionally responsible for product recalls due to food contamination.

*Staphylococcus aureus* is a Gram-positive, round-shaped bacterium that is a member of the Firmicutes, and it is a usual member of the microbiota of the body, frequently found in the upper respiratory tract and on the skin. It is often positive for catalase and nitrate reduction and is a facultative anaerobe that can grow without the need for oxygen. Skin infections are the most common form of *S. aureus* infection. This can manifest in various ways, including small benign boils, folliculitis, impetigo, cellulitis, and more severe, invasive soft-tissue infections.

*S. aureus* is extremely prevalent in persons with atopic dermatitis, more commonly known as eczema. It is mostly found in fertile, active places, including the armpits, hair, and scalp. Large pimples that appear in those areas may exacerbate the infection if lacerated. This can lead to staphylococcal scalded skin syndrome, a severe form of which can be seen in newborns.

*Staphylococcus epidermidis,* a Gram-positive bacterium, is a part of the normal human flora, typically the skin flora, and less commonly the mucosal flora. It is a facultative anaerobic bacteria.

As used herein, the term "SCFA Blend" is intended to refer to any combination of short chain fatty acids described herein.

Further tests of adding 0.6 wt. % SCFA Blend into a commercially available body wash and application thereof to a skin tissue, showed the body wash to upregulate hydration related gene and tight junction gene and downregulated inflammation related gene expressions. This revolutionary technology is suitable to body wash, lotion or underarm product to provide skin microbiome benefits.

The present invention is directed to a personal care composition comprising lactic acid, mandelic acid and pyruvic acid at about 4:2:1 ratio; and a surfactant. In other embodiments, the present invention provides a complex comprising a salt of lactic acid; a salt of mandelic acid; and a salt of pyruvic acid (e.g. sodium pyruvate).

One of the advantages is that the personal care composition provides for an improved skin microflora balance.

Another advantage is that the application of the personal care composition inhibits the growth of harmful bacteria such as *Escherichia coli, Corynebacterium striatum,* and *Staphylococcus aureus.*

Still another advantage is that the personal care composition promotes the growth of beneficial bacteria, such as *Staphylococcus epidermidis.*

A further advantage of personal care composition is the use of the composition upregulated AQP, FASN, OCLN gene expression.

Additional advantage of personal care composition is the use of the composition downregulated IL8 gene expression.

In some embodiments, the personal care compositions of the present invention improve skin barrier function and hydration. Without being bound by theory, the present inventors believe that the effects observed with the compositions described herein are the result of increasing filaggrin and occludin expression.

The present invention is directed to a personal care composition comprising a blend of short chain fatty acids ("SCFA Blend") as defined in the claims and a cosmetically acceptable carrier. In some embodiments, the cosmetically acceptable carrier comprises a surfactant. In some embodiments, the personal care composition further comprises an ingredient known to have antiperspirant or deodorant effect. A personal care composition is any composition that is applied to a person, either neat, or as a mixture. A personal care composition may be applied, for example, to the skin of the body, to the skin of the face, to the skin of the neck, to the lips, to the hair and scalp, to the appendages (e.g. hands, feet), auxiliary areas (e.g. armpits) or to the oral cavity.

Personal care compositions include hair care, skin care, sun care, facial care, nail care, and oral care compositions. In some embodiments, the personal care composition is applied to the person's skin or scalp. Examples of personal care compositions include an antiperspirant, a deodorant, a body wash, a shower gel, a lotion, a peel (e.g. a chemical peel), a bar soap, a soft soap, a shampoo, a hair conditioner, a sunscreen, a facial cleanser, a facial toner, an exfoliant, a mask, a serum, a moisturizer, and a cosmetic (e.g. lipstick, concealer, blush, eye shadow). The personal care composition may be defined, classified and/or regulated by national or international regulatory agencies as a cosmetic, or as a drug.

In some embodiments, the present invention provides a personal care composition comprising 4-butylresorcinol (e.g., from about 0.1 wt.% to about 5 wt.%, optionally about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, about 2 wt.%, about 3 wt.%, about 4 wt.% or about 5 wt.%, based on the total weight of the personal care composition). In other embodiments, the present invention provides a personal care composition comprising hexylresorcinol (e.g., from about 0.1 wt.% to about 5 wt.%, optionally about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, about 2 wt.%, about 3 wt.%, about 4 wt.% or about 5 wt.%, based on the total weight of the personal care composition). In further embodiments, the present invention provides a personal care composition comprising Silybum Marianum (milk thistle) extract (e.g., from about 0.01 wt.% to about 2 wt.%, optionally about 0.01 wt.%, about 0.05 wt.%, about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, or about 2 wt.%, based on the total weight of the personal care composition). Still further embodiments provide personal care compositions comprising dimethylmethoxy chromanol (e.g., from about 0.01 wt.% to about 1 wt.%, optionally about 0.01 wt.%, about 0.02 wt.%, about 0.03 wt.%, about 0.04 wt.%, about 0.05 wt.%, about 0.06 wt.%, about 0.07 wt.%, about 0.08 wt.%, about 0.09 wt.%, about 0.1 wt.%, about 0.5 wt.%, about 0.75 wt.%, or about 1 wt.%, based on the total weight of the personal care composition). Yet other embodiments provide personal care compositions comprising 4-butylresorcinol (e.g., from about 0.1 wt.% to about 5 wt.%, optionally about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, about 2 wt.%, about 3 wt.%, about 4 wt.% or about 5 wt.%, based on the total weight of the personal care composition); hexylresorcinol (e.g., from about 0.1 wt.% to about 5 wt.%, optionally about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, about 2 wt.%, about 3 wt.%, about 4 wt.% or about 5 wt.%, based on the total weight of the personal care composition); Silybum Marianum (milk thistle) extract (e.g., from about 0.01 wt.% to about 2 wt.%, optionally about 0.01 wt.%, about 0.05 wt.%, about 0.1 wt.%, about 0.2 wt.%, about 0.3 wt.%, about 0.4 wt.%, about 0.5 wt.%, about 0.6 wt.%, about 0.7 wt., about 0.8 wt.%, about 0.9 wt.%, about %1 wt.%, or about 2 wt.%, based on the total weight of the personal care composition); and dimethylmethoxy chromanol (e.g., from about 0.01 wt.% to about 1 wt.%, optionally about 0.01 wt.%, about 0.02 wt.%, about 0.03 wt.%, about 0.04 wt.%, about 0.05 wt.%, about 0.06 wt.%, about 0.07 wt.%, about 0.08 wt.%, about 0.09 wt.%, about 0.1 wt.%, about 0.5 wt.%, about 0.75 wt.%, or about 1 wt.%, based on the total weight of the personal care composition). In some embodiments, the personal care composition comprises any one of the complexes described herein comprising a plurality of short chain fatty acids, or salt thereof (e.g. lactic acid, mandelic acid and/or pyruvic acid or sodium pyruvate); 4-butylresorcinol; hexylresorcinol; Silybum Marianum (milk thistle) extract; and dimethylmethoxy chromanol, and is in the form of a peel (e.g., a chemical peel).

As used herein, "LMP" refers to a combination of lactic acid, mandelic acid and pyruvic acid.

As used herein, "LMP'" may refer to a combination of lactic acid, mandelic acid and sodium pyruvate.

As used herein, "MP" refers to a combination of mandelic acid and pyruvic acid.

As used herein, "MP'" may refer to a combination of mandelic acid and sodium pyruvate.

As used herein, "LP" refers to a combination of lactic acid and pyruvic acid.

As used herein, "LP'" may refer to a combination of lactic acid and sodium pyruvate.

As used herein, "LM" refers to a combination of lactic acid and mandelic acid.

A deodorant is a composition that is applied to the body of a person to prevent body odor caused by the bacterial breakdown of perspiration. A deodorant may be applied to any part of the body. Under selected embodiments, the deodorant is applicable to armpits and feet.

An antiperspirant is a composition that mitigates body odor as well as prevents sweating by affecting sweat glands. An antiperspirant may be applied to any part of the body, and are generally applied to the underarms.

A body wash is a liquid product used for cleaning the body during showers. A body wash comprises synthetic detergents derived from either petroleum or plant sources. A body wash has a lower pH value than soap, and is typically less drying to the skin than a soap.

Under one embodiment, a shower gel is synonymous with body wash. Under an alternative embodiment, a shower gel has a higher viscosity than body wash and has a more firm consistency. A shower gel may have an ingredient that has a cooling feel.

A lotion is a low-viscosity topical preparation for application to the skin. Lotions are applied to external skin with bare hands, a brush, or a clean cloth. A lotion, such as a hand lotion or a body lotion, provides smoothing, moisturizing, softening and perfuming of the skin. A lotion may be used as a medicine delivery system.

The personal care compositions of the present invention comprise a SCFA Blend comprising a plurality of short chain fatty acids ("SCFA") as defined in the claims. Optionally, the personal care composition may further comprise a short chained alcohol or a polyol.

As used herein, short-chain acids are acids with less than six carbon atoms. As used herein short-chain acids mean not only hydrocarbyl acids of formula CnH2n+1COOH, wherein n is 0 to 6, but also short-chained acids that are substituted with oxygen containing groups such as alcohols or oxo groups.

Short-chain acids that are hydrocarbyl acids are short-chain fatty acids. Examples of short-chain fatty acids include formic acid, methanoic acid, HCOOH, acetic acid, mandelic acid; ethanoic acid, CH₃COOH, propionic acid, CH₃CH₂COOH, butyric acid, butanoic acid, CH₃CH₂CH₂COOH, isobutyric acid, 2-methylpropanoic acid, (CH₃)₂CHCOOH, valeric acid, pentanoic acid, CH₃CH₂CH₂CH₂COOH, isovaleric acid, 3-methylbutanoic acid, and (CH₃)₂CHCH₂COOH.

Examples of short-chain acids comprising one carbon include methanoic acid, formic acid, and HCOOH.

Examples of short-chain acids comprising two carbons include ethanoic acid, acetic acid, CH₃COOH, trichloro acetic acid, thio acetic acid, ethanedioic acid, oxalic acid, HOOCCOOH, oxoethanoic acid, glyoxylic acid, formylformic acid, OHCCOOH, 2-hydroxyethanoic acid, glycolic acid, dicarbonous acid, hydroxyacetic acid, and HOCH₂COOH.

Examples of short-chain acids comprising three carbons include propanoic acid, ethanecarboxylic acid, CH₃CH₂COOH, prop-2-enoic acid, acrylic acid, acroleic acid, ethylenecarboxylic acid, propene acid, vinylformic acid, CH₂=CH-COOH, 2-propynoic acid, propiolic acid, acetylene carboxylic acid, propargylic acid, CH≡C-COOH, propanedioic acid, malonic acid, methanedicarboxylic acid, HOOC-CH₂-COOH, 2-hydroxypropanedioic acid, tartronic acid, hydroxymalonic acid, HOOC-CHOH-COOH, oxopropanedioic acid, mesoxalic acid, ketomalonic acid, HOOC-CO-COOH, 2,2-dihydroxypropanedioic acid, dihydroxymalonic acid, mesoxalic acid monohydrate, HOOC-C(OH)₂-COOH, 2-oxopropanoic acid, pyruvic acid, α-ketopropionic acid, acetylformic acid, pyroracemic acid, CH₃-CO-COOH, 2-hydroxypropanoic acid, lactic acid, milk acid, CH₃-CHOH-COOH, 3-hydroxypropanoic acid, hydracrylic acid, CH₂OH-CH₂-COOH, 2,3-dihydroxypropanoic acid, glyceric acid, CH₂OH-CHOH-COOH, 2-oxiranecarboxylic acid, and glycidic acid.

Examples of short-chain acids comprising four carbons include butanoic acid, butyric acid, propanecarboxylic acid, CH₃(CH₂)₂COOH, 2-methylpropanoic acid, isobutyric acid, isobutanoic acid, (CH₃)₂CHCOOH, 2-oxobutanoic acid, alpha-ketobutyric acid, CH₃-CH₂-CO-COOH, 3-oxobutanoic acid, acetoacetic acid, CH₃CO-CH₂-COOH, 4-oxobutanoic acid, succinic semialdehyde, HC(O)-CH₂-CH₂-COOH, (*E*)-butenedioic acid, fumaric acid, trans-1,2-ethylenedicarboxylic acid, 2-butenedioic acid, trans-butenedioic acid, allomaleic acid, boletic acid, donitic acid, lichenic acid, HOOC-CH=CH-COOH, (2)-butenedioic acid, maleic acid, cis-butenedioic acid, maleinic acid, toxilic acid, HOOC-CH=CH-COOH, oxobutanedioic aci, oxaloacetic acid, oxalacetic acid, oxosuccinic acid, HOOC-CH₂-CO-COOH, hydroxybutanedioic acid, malic acid, hydroxybutanedioic acid, HOOC-CH₂-CHOH-COOH, 2,3-dihydroxybutanedioic acid, tartaric acid, 2,3-dihydroxysuccinic acid, threaric acid, racemic acid, uvic acid, paratartaric acid, HOOC(CHOH)₂COOH, (*E*)-but-2-enoic acid, crotonic acid, trans-2-butenoic acid, beta-methylacrylic acid, 3-methylacrylic acid, (*E*)-2-butenoic acid, and CH₃-CH=CH-COOH.

Examples of short-chain acids comprising five carbons include pentanoic acid, valeric acid, valerianic acid, butane-1-carboxylic acid, CH₃(CH₂)₃COOH, 3-methylbutanoic acid, iso-valeric acid, (CH₃)₂CH-CH₂-COOH, pentanedioic acid, glutaric acid, propane-1,3-dicarboxylic acid, 1,3-propanedicarboxylic acid, *n*-pyrotartaric acid, HOOC-(CH₂)₃-COOH, 2-oxopentanedioic acid, alpha-ketoglutaric acid, 2-ketoglutaric acid, α-ketoglutaric acid, 2-oxoglutaric acid, oxoglutaric acid, and HOOC-(CH₂)₂-CO-COOH.

Under one embodiment, the personal care composition further comprises a short chain alcohol. Examples of a short chain alcohol includes methanol, CH₃OH, ethanol, CH₃CH₂OH, *n*-propanol, 1-propanol, CH₃-CH₂-CH₂-OH, iso-propanol, 2-propanol, (CH₃)₂CH-OH, *n*-butanol, 1-bunatol, CH₃-CH₂-CH₂-CH₂-OH, *sec*-butanol, 2-butanol, CH₃-CH₂-CHOH-CH₃, *iso*-butanol, (CH₃)₂CH-CH₂-OH, *tert*-butanol, (CH₃)₃C-OH, normal amyl alcohol, pentan-1-ol, CH₃-(CH₂)₄OH, isobutyl carbinol, 3-methylbutan-1-ol, isoamyl alcohol, isopentyl alcohol, (CH₃)₂CH-CH₂-CH₂-OH, active amyl alcohol, 2-methylbutan-1-ol, CH₃-CH₂-C(CH₃)H-CH₂-OH, tertiary butyl carbinol, 2,2-dimethylpropan-1-ol, neopentyl alcohol, (CH₃)₃C-CH₂-OH, 3-pentanol, pentan-3-ol, (CH₃-CH₂)CH-OH, methylpropyl carbinol, pentan-2-ol, CH₃-CH₂-CH₂-CH(OH)-CH₃, methyl isopropyl carbinol, 3-methylbutan-2-ol, (CH₃)₂CH-C(OH)-CH₃, dimethyl ethyl carbinol, 2-methylbutan-2-ol, tertiary amyl alcohol, CH₃-CH₂-C(CH₃)₂-OH.

Under one embodiment, the personal care composition further comprises a short chain polyol. A polyol is an organic compound containing multiple hydroxyl groups. Examples of polyols include a diol, a triol, and a tetrol. An example of a triol is a glycerol.

Lactic acid is a carboxylic acid with the formula CH3-CH(OH)-COOH. Under one embodiment, the lactic acid is L-(+)-lactic acid or (5)-lactic acid. Under another embodiment the lactic acid is D-(-)-lactic acid of (R)-lactic acid. Under yet another embodiment the lactic acid is a mixture of the two stereoisomers. In some embodiments, the lactic acid is a mixture of the two stereoisomers comprising at least 80% l-(+)-lactic acid or (5)-lactic acid. In another embodiments the lactic acid is a mixture of the two stereoisomers comprising at least 80% d-(-)-lactic acid or (R)-lactic acid.

Mandelic acid is a carboxylic acid with the formula C₆H₅CH(OH)CO₂H. Under one embodiment, the mandelic acid is S-(+)-mandelic acid or (*L*)- mandelic acid. Under another embodiment the mandelic acid is R-(-)-mandelic acid of or (D)- mandelic acid. Under yet another embodiment the mandelic acid is a mixture of the two stereoisomers. In another embodiment the mandelic acid is a mixture of the two stereoisomers comprising at least 80% S-(+)-mandelic acid or (*L*)-mandelic acid. In another embodiments, the mandelic acid is a mixture of the two stereoisomers comprising at least 80% R-(-)- mandelic acid or (D)-mandelic acid.

The personal care composition can comprise about 0.1 wt% to about 2.0 wt% of the SCFA Blend. Under one embodiment, the personal care composition comprises about 0.1 wt% to about 0.3 wt% of the SCFA Blend. Under one embodiment, the personal care composition comprises about 0.1 wt% to about 0.5 wt% of the SCFA Blend. Under one embodiment, the personal care composition comprises about 0.1 wt% to about 0.7 wt% of the SCFA Blend. Under one embodiment, the personal care composition comprises about 0.1 wt% to about 1.1 wt% of the SCFA Blend. Under one embodiment, the personal care composition comprises about 0.1 wt% to about 1.3 wt% of the SCFA Blend. Under one embodiment, the personal care composition comprises about 0.1 wt% to about 1.5 wt% of the SCFA Blend. Under one embodiment, the personal care composition comprises about 0.1 wt% to about 1.7 wt% of the SCFA Blend.

Under one embodiment, the personal care composition comprises about 0.3 wt% to about 0.5 wt% of the SCFA Blend. Under one embodiment, the personal care composition comprises about 0.3 wt% to about 0.7 wt% of the SCFA Blend. Under one embodiment, the personal care composition comprises about 0.3 wt% to about 1.1 wt% of the SCFA Blend. Under one embodiment, the personal care composition comprises about 0.3 wt% to about 1.3 wt% of the SCFA Blend. Under one embodiment, the personal care composition comprises about 0.3 wt% to about 1.5 wt% of the SCFA Blend. Under one embodiment, the personal care composition comprises about 0.3 wt% to about 1.7 wt% of the SCFA Blend. Under one embodiment, the personal care composition comprises about 0.3 wt% to about 2.0 wt% of the SCFA Blend.

Under one embodiment, the personal care composition comprises about 0.5 wt% to about 0.7 wt% of the SCFA Blend. Under one embodiment, the personal care composition comprises about 0.5 wt% to about 1.1 wt% of the SCFA Blend. Under one embodiment, the personal care composition comprises about 0.5 wt% to about 1.3 wt% of the SCFA Blend. Under one embodiment, the personal care composition comprises about 0.5 wt% to about 1.5 wt% of the SCFA Blend. Under one embodiment, the personal care composition comprises about 0.5 wt% to about 1.7 wt% of the SCFA Blend. Under one embodiment, the personal care composition comprises about 0.5 wt% to about 2.0 wt% of the SCFA Blend.

Under one embodiment, the personal care composition comprises about 0.7 wt% to about 1.1 wt% of the SCFA Blend. Under one embodiment, the personal care composition comprises about 0.7 wt% to about 1.3 wt% of the SCFA Blend. Under one embodiment, the personal care composition comprises about 0.7 wt% to about 1.5 wt% of the SCFA Blend. Under one embodiment, the personal care composition comprises about 0.7 wt% to about 1.7 wt% of the SCFA Blend. Under one embodiment, the personal care composition comprises about 0.7 wt% to about 2.0 wt% of the SCFA Blend.

Under one embodiment, the personal care composition comprises about 0.9 wt% to about 1.1 wt% of the SCFA Blend. Under one embodiment, the personal care composition comprises about 0.9 wt% to about 1.3 wt% of the SCFA Blend. Under one embodiment, the personal care composition comprises about 0.9 wt% to about 1.5 wt% of the SCFA Blend. Under one embodiment, the personal care composition comprises about 0.9 wt% to about 1.7 wt% of the SCFA Blend. Under one embodiment, the personal care composition comprises about 0.9 wt% to about 2.0 wt% of the SCFA Blend.

Under one embodiment, the personal care composition comprises about 1.1 wt% to about 1.3 wt% of the SCFA Blend. Under one embodiment, the personal care composition comprises about 1.1 wt% to about 1.5 wt% of the SCFA Blend. Under one embodiment, the personal care composition comprises about 1.1 wt% to about 1.7 wt% of the SCFA Blend. Under one embodiment, the personal care composition comprises about 1.1 wt% to about 2.0 wt% of the SCFA Blend.

Under one embodiment, the personal care composition comprises about 1.3 wt% to about 1.5 wt% of the SCFA Blend. Under one embodiment, the personal care composition comprises about 1.3 wt% to about 1.7 wt% of the SCFA Blend. Under one embodiment, the personal care composition comprises about 1.3 wt% to about 2.0 wt% of the SCFA Blend.

Under one embodiment, the personal care composition comprises about 1.5 wt% to about 1.7 wt% of the SCFA Blend. Under one embodiment, the personal care composition comprises about 1.5 wt% to about 2.0 wt% of the SCFA Blend. Under one embodiment, the personal care composition comprises about 1.7 wt% to about 2.0 wt% of the SCFA Blend.

The personal care product of the present invention comprises a SCFA Blend of short chain fatty acids, wherein the short chain fatty acids are lactic acid, mandelic acid, and pyruvic acid. The lactic is the major component, and the pyruvic acid is the smallest component. Under one embodiment, the SCFA Blend comprises about 2 to about 16 parts by weight lactic acid, about 1 to about 8 parts by weight mandelic acid, and about 0.5 to about 4 parts by weight pyruvic acid. The phrase "parts by weight" refers to the weight ratios of the component short chain fatty acids.

As defined in claim 1, the lactic acid, or a salt thereof, mandelic acid, or a salt thereof; and pyruvic acid, or a salt thereof are present in a weight ratio of about 4 : 2 : 1. Generally, another SCFA Blend can also comprise about 4 to about 12 parts by weight lactic acid, about 2 to about 6 parts by weight mandelic acid, and about 1 to about 3 parts by weight pyruvic acid. Another SCFA Blend can also comprise about 8 to about 12 parts by weight lactic acid, about 4 to about 5 parts by weight mandelic acid, and about 2 to about 2.5 parts by weight pyruvic acid. Another SCFA Blend can also comprise about 8 to about 14 parts by weight lactic acid, about 4 to about 5 parts by weight mandelic acid, and about 2.0 to about 2.5 parts by weight pyruvic acid. Another SCFA Blend can also comprise about 10 to about 12 parts by weight lactic acid, about 4 to about 5 parts by weight mandelic acid, and about 2.0 to about 2.5 parts by weight pyruvic acid. Another SCFA Blend can also comprise about 10 to about 14 parts by weight lactic acid, about 4 to about 5 parts by weight mandelic acid, and about 2.0 to about 2.5 parts by weight pyruvic acid. Another SCFA Blend can also comprise about 12 to about 14 parts by weight lactic acid, about 4 to about 5 parts by weight mandelic acid, and about 2.0 to about 2.5 parts by weight pyruvic acid.

Another SCFA Blend can also comprise about 8 to about 10 parts by weight lactic acid, about 4 to about 6 parts by weight mandelic acid, and about 2.0 to about 2.5 parts by weight pyruvic acid. Another SCFA Blend can also comprise about 8 to about 12 parts by weight lactic acid, about 4 to about 6 parts by weight mandelic acid, and about 2.0 to about 2.5 parts by weight pyruvic acid. Another SCFA Blend can also comprise about 8 to about 14 parts by weight lactic acid, about 4 to about 6 parts by weight mandelic acid, and about 2.0 to about 2.5 parts by weight pyruvic acid. Another SCFA Blend can also comprise about 10 to about 12 parts by weight lactic acid, about 4 to about 6 parts by weight mandelic acid, and about 2.0 to about 2.5 parts by weight pyruvic acid. Another SCFA Blend can also comprise about 10 to about 14 parts by weight lactic acid, about 4 to about 6 parts by weight mandelic acid, and about 2.0 to about 2.5 parts by weight pyruvic acid. Another SCFA Blend can also comprise about 12 to about 14 parts by weight lactic acid, about 4 to about 6 parts by weight mandelic acid, and about 2.0 to about 2.5 parts by weight pyruvic acid.

Another SCFA Blend can also comprise about 8 to about 10 parts by weight lactic acid, about 5 to about 6 parts by weight mandelic acid, and about 2.0 to about 2.5 parts by weight pyruvic acid. Another SCFA Blend can also comprise about 8 to about 12 parts by weight lactic acid, about 5 to about 6 parts by weight mandelic acid, and about 2.0 to about 2.5 parts by weight pyruvic acid. Another SCFA Blend can also comprise about 8 to about 14 parts by weight lactic acid, about 5 to about 6 parts by weight mandelic acid, and about 2.0 to about 2.5 parts by weight pyruvic acid. Another SCFA Blend can also comprise about 10 to about 12 parts by weight lactic acid, about 5 to about 6 parts by weight mandelic acid, and about 2.0 to about 2.5 parts by weight pyruvic acid. Another SCFA Blend can also comprise about 10 to about 14 parts by weight lactic acid, about 5 to about 6 parts by weight mandelic acid, and about 2.0 to about 2.5 parts by weight pyruvic acid. Another SCFA Blend can also comprise about 12 to about 14 parts by weight lactic acid, about 5 to about 6 parts by weight mandelic acid, and about 2.0 to about 2.5 parts by weight pyruvic acid.

Another SCFA Blend can also comprise about 8 to about 10 parts by weight lactic acid, about 4 to about 5 parts by weight mandelic acid, and about 2.0 to about 3.0 parts by weight pyruvic acid. Another SCFA Blend can also comprise about 8 to about 12 parts by weight lactic acid, about 4 to about 5 parts by weight mandelic acid, and about 2.0 to about 3.0 parts by weight pyruvic acid. Another SCFA Blend can also comprise about 8 to about 14 parts by weight lactic acid, about 4 to about 5 parts by weight mandelic acid, and about 2.0 to about 3.0 parts by weight pyruvic acid. Another SCFA Blend can also comprise about 10 to about 12 parts by weight lactic acid, about 4 to about 5 parts by weight mandelic acid, and about 2.0 to about 3.0 parts by weight pyruvic acid. Another SCFA Blend can also comprise about 10 to about 14 parts by weight lactic acid, about 4 to about 5 parts by weight mandelic acid, and about 2.0 to about 3.0 parts by weight pyruvic acid. Another SCFA Blend can also comprise about 12 to about 14 parts by weight lactic acid, about 4 to about 5 parts by weight mandelic acid, and about 2.0 to about 3.0 parts by weight pyruvic acid.

Another SCFA Blend can also comprise about 8 to about 10 parts by weight lactic acid, about 4 to about 6 parts by weight mandelic acid, and about 2.0 to about 3.0 parts by weight pyruvic acid. Another SCFA Blend can also comprise about 8 to about 12 parts by weight lactic acid, about 4 to about 6 parts by weight mandelic acid, and about 2.0 to about 3.0 parts by weight pyruvic acid. Another SCFA Blend can also comprise about 8 to about 14 parts by weight lactic acid, about 4 to about 6 parts by weight mandelic acid, and about 2.0 to about 3.0 parts by weight pyruvic acid. Another SCFA Blend can also comprise about 10 to about 12 parts by weight lactic acid, about 4 to about 6 parts by weight mandelic acid, and about 2.0 to about 3.0 parts by weight pyruvic acid. Another SCFA Blend can also comprise about 10 to about 14 parts by weight lactic acid, about 4 to about 6 parts by weight mandelic acid, and about 2.0 to about 3.0 parts by weight pyruvic acid. Another SCFA Blend can also comprise about 12 to about 14 parts by weight lactic acid, about 4 to about 6 parts by weight mandelic acid, and about 2.0 to about 3.0 parts by weight pyruvic acid.

Another SCFA Blend can also comprise about 8 to about 10 parts by weight lactic acid, about 5 to about 6 parts by weight mandelic acid, and about 2.0 to about 3.0 parts by weight pyruvic acid. Another SCFA Blend can also comprise about 8 to about 12 parts by weight lactic acid, about 5 to about 6 parts by weight mandelic acid, and about 2.0 to about 3.0 parts by weight pyruvic acid. Another SCFA Blend can also comprise about 8 to about 14 parts by weight lactic acid, about 5 to about 6 parts by weight mandelic acid, and about 2.0 to about 3.0 parts by weight pyruvic acid. Another SCFA Blend can also comprise about 10 to about 12 parts by weight lactic acid, about 5 to about 6 parts by weight mandelic acid, and about 2.0 to about 3.0 parts by weight pyruvic acid. Another SCFA Blend can also comprise about 10 to about 14 parts by weight lactic acid, about 5 to about 6 parts by weight mandelic acid, and about 2.0 to about 3.0 parts by weight pyruvic acid. Another SCFA Blend can also comprise about 12 to about 14 parts by weight lactic acid, about 5 to about 6 parts by weight mandelic acid, and about 2.0 to about 3.0 parts by weight pyruvic acid.

Another SCFA Blend can also comprise about 8 to about 10 parts by weight lactic acid, about 4 to about 5 parts by weight mandelic acid, and about 2.5 to about 3.0 parts by weight pyruvic acid. Another SCFA Blend can also comprise about 8 to about 12 parts by weight lactic acid, about 4 to about 5 parts by weight mandelic acid, and about 2.5 to about 3.0 parts by weight pyruvic acid. Another SCFA Blend can also comprises about 8 to about 14 parts by weight lactic acid, about 4 to about 5 parts by weight mandelic acid, and about 2.5 to about 3.0 parts by weight pyruvic acid. Another SCFA Blend can also comprises about 10 to about 12 parts by weight lactic acid, about 4 to about 5 parts by weight mandelic acid, and about 2.5 to about 3.0 parts by weight pyruvic acid. Another SCFA Blend can also comprises about 10 to about 14 parts by weight lactic acid, about 4 to about 5 parts by weight mandelic acid, and about 2.5 to about 3.0 parts by weight pyruvic acid. Another SCFA Blend can also comprise about 12 to about 14 parts by weight lactic acid, about 4 to about 5 parts by weight mandelic acid, and about 2.5 to about 3.0 parts by weight pyruvic acid.

Another SCFA Blend can also comprise about 8 to about 10 parts by weight lactic acid, about 4 to about 6 parts by weight mandelic acid, and about 2.5 to about 3.0 parts by weight pyruvic acid. Another SCFA Blend can also comprise about 8 to about 12 parts by weight lactic acid, about 4 to about 6 parts by weight mandelic acid, and about 2.5 to about 3.0 parts by weight pyruvic acid. Another SCFA Blend can also comprise about 8 to about 14 parts by weight lactic acid, about 4 to about 6 parts by weight mandelic acid, and about 2.5 to about 3.0 parts by weight pyruvic acid. Another SCFA Blend can also comprise about 10 to about 12 parts by weight lactic acid, about 4 to about 6 parts by weight mandelic acid, and about 2.5 to about 3.0 parts by weight pyruvic acid. Another SCFA Blend can also comprise about 10 to about 14 parts by weight lactic acid, about 4 to about 6 parts by weight mandelic acid, and about 2.5 to about 3.0 parts by weight pyruvic acid. Another SCFA Blend can also comprise about 12 to about 14 parts by weight lactic acid, about 4 to about 6 parts by weight mandelic acid, and about 2.5 to about 3.0 parts by weight pyruvic acid.

Another SCFA Blend can also comprise about 8 to about 10 parts by weight lactic acid, about 5 to about 6 parts by weight mandelic acid, and about 2.5 to about 3.0 parts by weight pyruvic acid. Another SCFA Blend can also comprise about 8 to about 12 parts by weight lactic acid, about 5 to about 6 parts by weight mandelic acid, and about 2.5 to about 3.0 parts by weight pyruvic acid. Another SCFA Blend can also comprise about 8 to about 14 parts by weight lactic acid, about 5 to about 6 parts by weight mandelic acid, and about 2.5 to about 3.0 parts by weight pyruvic acid. Another SCFA Blend can also comprise about 10 to about 12 parts by weight lactic acid, about 5 to about 6 parts by weight mandelic acid, and about 2.5 to about 3.0 parts by weight pyruvic acid. Another SCFA Blend can also comprise about 10 to about 14 parts by weight lactic acid, about 5 to about 6 parts by weight mandelic acid, and about 2.5 to about 3.0 parts by weight pyruvic acid. Another SCFA Blend can also comprise about 12 to about 14 parts by weight lactic acid, about 5 to about 6 parts by weight mandelic acid, and about 2.5 to about 3.0 parts by weight pyruvic acid.

Another SCFA Blend can also comprise about 8 to about 14 parts by weight lactic acid, about 4 to about 6 parts by weight mandelic acid, and about 2.0 to about 3.0 parts by weight pyruvic acid, and about 1 parts of glycerol.

In some embodiments, the complexes described herein may comprise a SCFA Blend alone, or a SCFA Blend in combination with other ingredients.

In some embodiments, the personal care composition includes a surfactant. The surfactant can be any anionic, nonionic, amphoteric, or zwitterionic surfactant, or combinations thereof. The amount of surfactant in the composition is at least 1 weight %. In other embodiments, the amount is 1 to 20 weight %, or at least 2, 3, 4, 5, 6, 7, 8, 9, or 10 weight %.

Under various embodiments, surfactants make up 10, 20, 30, 40, or 50 percent of the personal care composition. Under an embodiment, multiple surfactants are used to achieve desired product qualities. A primary surfactant provides good foaming ability and cleaning effectiveness, while a secondary surfactant adds qualities of mildness to prevent irritation or over-drying of the skin. To prevent ingredients from separating, emulsifiers such as diethanolamine may be added. Additional ingredients include conditioning agents that moisturize the skin during and after product use. Ingredients, like scent in the form of essential oils or fragrance oils, and colorant in the form of water soluble dyes may also be used.

A variety of anionic surfactants can be utilized in the personal care composition including, for example, long chain alkyl (C₆-C₂₂) materials such as long chain alkyl sulfates, long chain alkyl sulfonates, long chain alkyl phosphates, long chain alkyl ether sulfates, long chain alkyl alpha olefin sulfonates, long chain alkyl taurates, long chain alkyl isethionates (SCI), long chain alkyl glyceryl ether sulfonates (AGES), sulfosuccinates. These anionic surfactants can be alkoxylated, for example, ethoxylated, although alkoxylation is not required. These surfactants are typically highly water soluble as their sodium, potassium, alkyl and ammonium or alkanol ammonium containing salt form and can provide high foaming cleansing power. Other equivalent anionic surfactants may be used. In one embodiment, the anionic surfactant comprises sodium laureth sulfate, sodium pareth sulfate, and combinations thereof. Anionic surfactants can be included in any desired amount. In one embodiment, anionic surfactants are present in the composition in an amount of 0 to about 15% by weight. In one embodiment, anionic surfactants are present in an amount of about 6 to about 8% by weight.

Amphoteric surfactants may also be included in the composition. These surfactants are typically characterized by a combination of high surfactant activity, lather forming and mildness. Amphoteric surfactants include, but are not limited to derivatives of aliphatic secondary and tertiary amines in which the aliphatic radical can be straight chain or branched and wherein one of the aliphatic substituents contains about 8 to about 18 carbon atoms and one contains an anionic water solubilizing group, e.g., carboxy, sulfonate, sulfate, phosphate, or phosphonate. Examples of such compounds include sodium 3-dodecyaminopropionate, sodium 3-dodecylaminopropane sulfonate, N-alkyl taurines and N-higher alkyl aspartic acids. Other equivalent amphoteric surfactants may be used. Examples of amphoteric surfactants include, but are not limited to, a range of betaines including, for example, high alkyl betaines, such as coco dimethyl carboxymethyl betaine, lauryl dimethyl carboxy-methyl betaine, lauryl dimethyl alpha-carboxyethyl betaine, cetyl dimethyl carboxymethyl betaine, lauryl bis-(2-hydroxyethyl)carboxy methyl betaine, stearyl bis-(2-hydroxypropyl)carboxymethyl betaine, oleyl dimethyl gamma-carboxypropyl betaine, and lauryl bis-(2-hydroxypropyl)alpha-carboxyethyl betaine, sulfobetaines such as coco dimethyl sulfopropyl betaine, stearyl dimethyl sulfopropyl betaine, amido betaines, amidosulfobetaines. Betaines having a long chain alkyl group, particularly coco, may be particularly useful as are those that include an amido groups such as the cocamidopropyl and cocoamidoethyl betaines. Amphoteric surfactants can be included in any desired amount. In one embodiment, amphoteric surfactants are present in the composition in an amount of 0 to about 15% by weight. In one embodiment, the amphoteric surfactants are present in the composition in an amount of about 4 to about 6% by weight.

Examples of nonionic surfactants include polysorbate 20, long chain alkyl glucosides having C₈-C₂₂ alkyl groups; coconut fatty acid monoethanolamides such as cocamide MEA; coconut fatty acid diethanolamides, fatty alcohol ethoxylates (alkylpolyethylene glycols); alkylphenol polyethylene glycols; alkyl mercaptan polyethylene glycols; fatty amine ethoxylates (alkylaminopolyethylene glycols); fatty acid ethoxylates (acyl polyethylene glycols); polypropylene glycol ethoxylates (for example the PLURONIC^{™} block copolymers commercially available from BASF); fatty acid alkylolamides, (fatty acid amide polyethylene glycols); N-alkyl-, N-alkoxypolyhydroxy fatty acid amides; sucrose esters; sorbitol esters; polyglycol ethers; and combinations thereof. Nonionic surfactants can be included in any desired amount. In one embodiment, nonionic surfactants are present in the composition in an amount of 0 to about 3% by weight. In one embodiment, nonionic surfactants are present in the composition in an amount of about 0.5 to about 1.5% by weight.

Cationic surfactants can also be included in the composition. Examples of cationic surfactants include any quaternium or polyquaternium compound. Cationic surfactants can be included at any desired level. In one embodiment, cationic surfactants are present in the composition in an amount of 0 to about 2% by weight. In one embodiment, cationic surfactants are present in the composition in an amount of about 0.1 to about 0.3% by weight.

Additional ingredients may be present in the personal care composition. These include water and ingredients to thicken, preserve, emulsify, add fragrance, and color.

Skin compatible oils can be included in the composition. Skin compatible oils include a range of liquid hydrocarbons, for example, linear and branched oils such as liquid paraffin, squalene, squalane, mineral oil, low viscosity synthetic hydrocarbons such as polyalphaolefins, commercially available from ExxonMobil under the trade name PURESYN PAO and polybutene under the trade name PANALANE^{™} or INDOPOL^{™}. Light (low viscosity) highly branched hydrocarbon oils may also be suitable in some instances. Other useful skin compatible oils may be silicone based, for example, linear and cyclic polydimethyl siloxane, organo functional silicones (alkyl and alkyl aryl), and amino silicones.

In other embodiments, the composition may include any of following materials in any desired amount to achieve a desired effect in the composition (amounts that can be used in some embodiments are provided): one or more alkaline salts, for example, sodium chloride, sodium sulfate, sodium carbonate, sodium bicarbonate and/or their equivalents (0 to 5% by weight); foaming agents, for example decyl glucoside, and/or their equivalents (0 to 3% by weight); glyceryl esters and derivatives, for example glycol distearate, and/or their equivalents (0 to 3%; by weight); sequestrants, for example, tetrasodium EDTA, and/or their equivalents (0 to 2% by weight); biocides, for example, Triclosan (2,4,4'-trichloro-2'-hydroxydiphenyl ether), DMDM hydantoin, formaldehyde and/or imidazolidinyl urea, and/or their equivalents (0 to 2% by weight); organic acids, for example, citric acid and/or formic acid and/or their equivalents (0 to 2% by weight); viscosity modifiers (0 to 2% by weight); fragrances and/or perfumes (0 to 5% by weight); preservatives, for example, phenoxyethanol, formaldehyde solution, parabens, pentanediol or sorbic acid (0 to 2% by weight); pearlizing agents, for example, glycol distearic esters, such as ethylene glycol distearate, but also fatty acid monoglycol esters (0 to 3% by weight); stabilizers, for example, metal salts of fatty acids, such as e.g. magnesium stearate, aluminum stearate and/or zinc stearate (0 to 2% by a weight); and dyes and pigments that are approved and suitable for cosmetic purposes.

Water may be included in the composition. Water can be included in an amount of 0 to about 90% by weight. In one embodiment, water is present at about 50% to about 90% by weight.

In one embodiment, a moisturizing body wash composition also utilizes, as a thickening agent, a blend of PEG-150 distearate and PPG-2 hydroxyethyl cocamide for countering a decrease in viscosity associated with the concentrations of moisturizing agents utilized in some embodiments of the moisturizing body wash composition. This blended thickening agent allows the composition to achieve viscosities beyond those that could be achieved with conventional thickening agents, for example sodium chloride alone, and is able to achieve suitable viscosities at relatively low concentrations. The relatively low concentrations used to achieve the desired viscosities are also advantageous with respect to manufacturing processes that may be employed to manufacture the moisturizing body wash composition, thereby reducing the need for larger equipment or modifications and the capital expenditure associated with manufacturing the moisturizing body wash composition if other thickening agents were used. The PEG-150 distearate and the PPG-2 hydroxyethyl cocamide can be present in any amount to achieve a desired viscosity. In one embodiment, the amount of PEG-150 distearate in the composition is 0 to about 2% by weight. In one embodiment, the amount of PPG-2 hydroxyethyl cocamide in the composition is 0 to about 2% by weight. In one embodiment, the weight ratio of the PEG-150 distearate to the PPG-2 hydroxyethyl cocamide can be about 3:1 to about 1:3. In one embodiment the PEG-150 distearate and the PPG-2 hydroxyethyl cocamide are each present at 0.0225% by weight. The PEG-150 distearate and the PPG-2 hydroxyethyl cocamide are available as a mixture from Uniqema under the trade name PROMIDIUM^{™} LTS.

Microbeads may also be added to the personal care composition. Microbeads are microspheres that may added to a variety of cosmetic products for their exfoliating qualities.

Personal care composition may also contain the ingredient menthol, which gives a cooling and stimulating sensation on the skin, and some men's shower gels are also designed specifically for use on hair and body. Shower gels contain milder surfactant bases than shampoos, and some also contain gentle conditioning agents in the formula. This means that shower gels can also double as an effective and perfectly acceptable substitute to shampoo, even if they are not labelled as a hair and body wash. Washing hair with shower gel should give approximately the same result as using a moisturizing shampoo

Also disclosed is a method of treating a skin of a patient in need thereof, the method comprising administering to the skin the above described composition, effective to promote the growth of beneficial bacteria and inhibit the growth of harmful bacteria, wherein the beneficial bacterium is selected from the group consisting of S. epidermidis, and harmful bacteria is selected from the group consisting of E. coli, C. straitum, and S. aureus.

The composition is spread on the skin neat, or it may be admixed with water. The spreading of the personal care composition may be done by hand, or it may be done by a instrument such as a glove or a piece of cloth.

The method of applying the personal care composition leaves behind a film. The thickness of the film depends on other parts of the formulation, but for a lotion, the thickness will be comparable to the thicknesses of other lotions, body washes, or deodorants. For those embodiments wherein the personal care composition is a lotion, the thickness may be about 50 µm.

The composition can be used in a method of treating a skin of a patient, wherein the amount of harmful bacteria is reduced by about 10%, as measured after 48 hours on a 50 µm thick layer of personal care composition comprising 0.6 wt% SCFA Blend, compared to the personal care composition not comprising the SCFA Blend. The amount of harmful bacteria can be reduced by at least about 10%, as measured after 48 hours on a 50 µm thick layer of personal care composition comprising 0.6 wt% SCFA Blend, compared to the personal care composition not comprising the SCFA Blend.

In a method of treating a skin of a patient, the amount of beneficial bacteria can be increased by about 10%, as measured after 48 hours on a 50 µm thick layer of personal care composition comprising 0.6 wt% SCFA Blend, compared to the personal care composition not comprising the SCFA Blend. The amount of beneficial bacteria can be increased by at least about 10%, as measured after 48 hours on a 50 µm thick layer of personal care composition comprising 0.6 wt% SCFA Blend, compared to the personal care composition not comprising the SCFA Blend.

In other embodiments, the personal care composition does not contain acetic acid.

As used herein, the term "substantially free" is intended to mean a negligible amount, for example, less than about 3 wt.% of the composition, and preferably less than 1 wt.% of the composition.

The first short chain fatty acid and the second short chain fatty acid are present in a weight ratio of about 2 : 1.

The weight ratio of the first short chain fatty acid (e.g., lactic acid) to the second short chain fatty acid (e.g. mandelic acid) is about 2 : 1. The weight ratio of the first short chain fatty acid (e.g. lactic acid) to the third short chain fatty acid (e.g., pyruvic acid) is about 4 : 1.

A method of increasing the firmness and/or elasticity of the skin of a patient in need thereof, can be comprising administering to the skin of said patient, an effective amount of a personal care composition comprising: a cosmetically acceptable carrier; and a blend of three short chain fatty acids as defined in the claims.

A method for inhibiting: tyrosinase; collagenase; and/or elastase, in a subject in need thereof, can be comprising administering to the skin of said subject, an effective amount of a personal care composition comprising: a cosmetically acceptable carrier; and a blend of three short chain fatty acids as defined in the claims.

In some embodiments, the present invention provides personal care compositions that are substantially free of hydroquinone. In other embodiments, the present invention provides personal care compositions that are free of hydroquinone.

The first short chain fatty acid comprises lactic acid, or a salt thereof. The second short chain fatty acid comprises mandelic acid, or a salt thereof. The third short chain fatty acid comprises pyruvic acid, or a salt thereof. The first short chain fatty acid comprises lactic acid, or a salt thereof; the second short chain fatty acid comprises mandelic acid, or a salt thereof; and the third short chain fatty acid comprises pyruvic acid, or a salt thereof.

Generally, a personal care composition can be comprising: from about 2 to about 20 wt.% lactic acid, or a salt thereof; from about 2 to about 12 wt.% mandelic acid, or a salt thereof; and from about 0.5 to about 8 wt.% pyruvic acid, or a salt thereof. Other personal care compositions can be comprising: from about 4 to about 18 wt.% lactic acid, or a salt thereof; from about 4 to about 10 wt.% mandelic acid, or a salt thereof; and from about 0.5 to about 6 wt.% pyruvic acid, or a salt thereof.

Generally, a personal care composition can be comprising: about 16 wt.% lactic acid, or a salt thereof; about 8 wt.% mandelic acid, or a salt thereof; and about 4 wt.% pyruvic acid, or a salt thereof. Generally, a personal care composition can be comprising: about 4 wt.% lactic acid, or a salt thereof; about 2 wt.% mandelic acid, or a salt thereof; and about 1 wt.% pyruvic acid, or a salt thereof.

Some embodiments of the present invention provide a personal care composition comprising: about 16 wt.% lactic acid; about 8 wt.% mandelic acid; about 4 wt.% pyruvic acid; from about 0.1 wt.% to about 3 wt.% of a tyrosinase inhibitor (e.g. 4-butylresorcinol); from about 0.1 wt.% to about 3 wt.% of a tyrosinase & peroxidase inhibitor (e.g. hexylresorcinol); from about 0.1 wt.% to about 2 wt.% of an antioxidant (e.g. silymarin); and/or a tocopherol analogue (e.g. dimethylmethoxy chromanol). In some embodiments, the personal care compositions described herein further comprise from about 0.1 wt.% to about 5 wt.% of an anti-inflammatory agent (e.g. dimethyl sulfone); from about 0.1 wt.% to about 5 wt.% acetyl glycyl beta-alanin; and/or a melanin synthesis inhibitor.

The first short chain fatty, the second short chain fatty acid and the third short chain fatty acid are present in a weight ratio of about 4 : 2 : 1. The personal care composition as defined in the claims is comprising lactic acid, or a salt thereof, mandelic acid, or a salt thereof; and pyruvic acid, or a salt thereof, in a weight ratio of about 4 : 2 : 1.

A method of treating, preventing or ameliorating a symptom associated with a disease, disorder or condition of the skin, can be comprising administering to the skin of a subject in need thereof, an effective amount of any one of the personal care compositions described herein. The effective amount can be an amount capable of promoting the growth of beneficial bacteria and/or inhibiting the growth of harmful bacteria. The beneficial bacteria can comprise *S. epidermidis.* The harmful bacteria can comprise: *E. coli, C. straitum,* and/or *S. aureus.* The amount of harmful bacteria can be reduced by at least about 10%. The amount of beneficial bacteria can be increased by at least about 10%.

A method of increasing the firmness and/or elasticity of the skin of a patient in need thereof, can be comprising administering to the skin of said patient, an effective amount of any one of the personal care compositions described herein.

A method for inhibiting: tyrosinase; collagenase; and/or elastase in a patient in need thereof, can be comprising administering to the skin of said patient, an effective amount of any one of the personal care compositions described herein.

For avoidance of doubt, salts of any one of the short chain fatty acids described herein, would be suitable for use in the personal care compositions of the present invention.

### EXAMPLES

Examples which do not fall under the scope of the claims are comparative examples.

### Example 1

A study of tyrosinase inhibition is conducted (e.g., Sigma Aldrich MAK 257 Kit) to investigate the efficacy of an exemplary composition of the present invention (0.312% lactic acid; 0.156% mandelic acid; and 0.078% pyruvic acid) and individual short chain fatty acids at the same concentrations.

**Table A**

| **Sample** | **Wt.%** | **% Tyrosinase Inhibition** |
|---|---|---|
| Lactic Acid (L) | 0.312 | 2.23 |
| Mandelic Acid (M) | 0.156 | 0.06 |
| Pyruvic Acid (P) | 0.078 | 0.37 |
| L + M + P | 0.312 (L) + 0.156 (M) + 0.078 (P) | 21.93 |

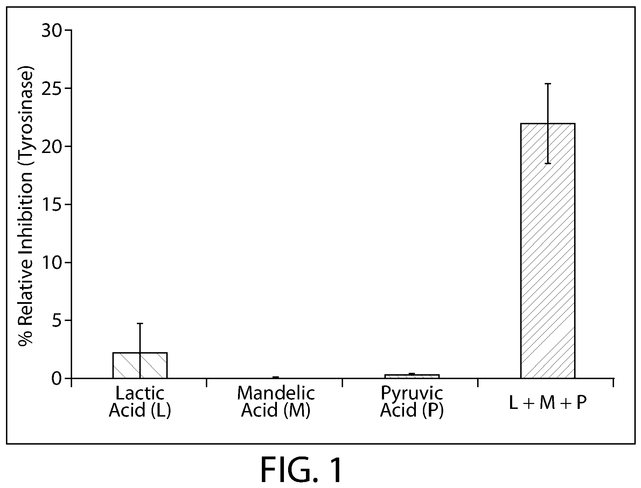

As described by the data reported in Figure 1 a strong synergy between lactic acid, mandelic acid and pyruvic acid was confirmed. The raw data that generated the bar graph depicted in Figure 1, is described above in Table A.

### Example 2

A study of collagenase inhibition is conducted (e.g., Sigma Aldrich MAK 293 Kit) to investigate the was conducted to investigate the efficacy of an exemplary composition of the present invention (1.25% lactic acid; 0.625% mandelic acid; and 0.312% pyruvic acid) and individual short chain fatty acids, at the same concentrations.

**Table B**

| **Sample** | **Wt.%** | **% Collagenase Inhibition** |
|---|---|---|
| Lactic Acid (L) | 1.25 | 12.71 |
| Mandelic Acid (M) | 0.625 | 8.57 |
| Pyruvic Acid (P) | 0.312 | 6.70 |
| L + M + P | 1.25 (L) + 0.625 (M) + 0.312 (P) | 48.84 |

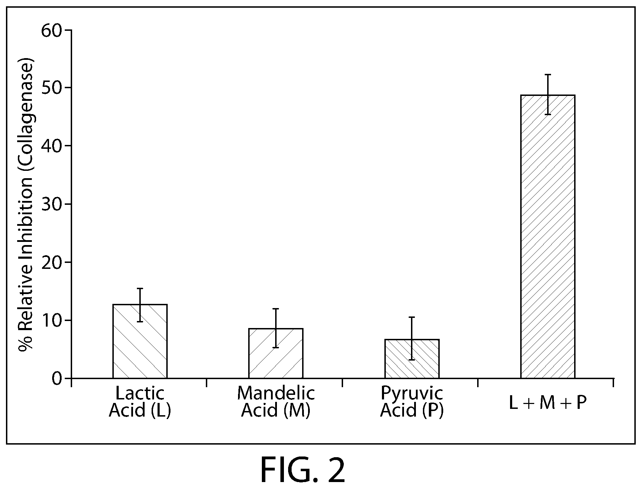

As described by the data reported in Figure 2 a strong synergy between lactic acid, mandelic acid and pyruvic acid was confirmed. The raw data that generated the bar graph depicted in Figure 2, is described above in Table B.

### Example 3 (comparative example)

A study of tyrosinase inhibition is conducted (e.g., Sigma Aldrich MAK 257 Kit) to investigate the efficacy of a comparative composition (0.312% mandelic acid and 0.156% pyruvic acid) and individual short chain fatty acids at the same concentrations.

**Table C**

| **Sample** | **Wt.%** | **% Tyrosinase Inhibition** |
|---|---|---|
| Mandelic Acid (M) | 0.312 | 1.17 |
| Pyruvic Acid (P) | 0.156 | 26.62 |
| M + P | 0.312 (M) + 0.156 (P) | 92.51 |

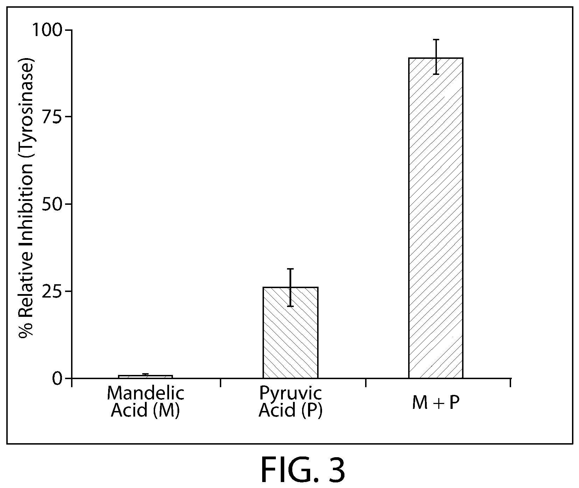

As described by the data reported in Figure 3 a strong synergy between mandelic acid and pyruvic acid was confirmed. The raw data that generated the bar graph depicted in Figure 3, is described above in Table C.

### Example 4 (comparative example)

A study of collagenase inhibition is conducted (e.g., Sigma Aldrich MAK 293 Kit) to investigate the was conducted to investigate the efficacy of a comparative composition (1.25% mandelic acid and 0.625% pyruvic acid) and individual short chain fatty acids, at the same concentrations.

**Table D**

| **Sample** | **Wt.%** | **% Collagenase Inhibition** |
|---|---|---|
| Mandelic Acid (M) | 1.25 | 11.26 |
| Pyruvic Acid (P) | 0.625 | 9.58 |
| M + P | 1.25 (M) + 0.625 (P) | 27.87 |

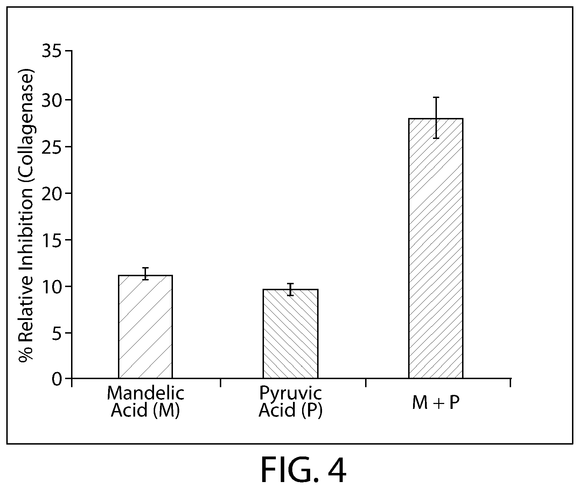

As described by the data reported in Figure 4 a strong synergy between mandelic acid and pyruvic acid was confirmed. The raw data that generated the bar graph depicted in Figure 4, is described above in Table D.

### Example 5 (comparative composition)

The anti-tyrosinase activity of a comparative composition was compared to the anti-tyrosinase activity of individual short chain fatty acids. Assays were performed using a tyrosinase inhibitor screening kit from Sigma Aldrich (MAK 257 Kit); and following the manufacturer's protocol. The results of these experiments are described in Table 1 (below).

**Table 1**

| **Sample** | **Wt.%** | **% Tyrosinase Inhibition** |
|---|---|---|
| Lactic Acid (L) | 0.312 | 52.72 |
| Pyruvic Acid (P) | 0.078 | 29.29 |
| L + P | 0.312 (L) + 0.078 (P) | 92.89 |

As demonstrated by the data described in Table 1 (above), a comparative composition provides synergistic inhibition of tyrosinase.

### Example 6 (comparative composition)

The anti-collagenase activity of a comparative composition was compared to the anti-collagenase activity of individual short chain fatty acids. Assays were performed using a collagenase inhibitor screening kit from Sigma Aldrich (MAK 293 Kit); and following the manufacturer's protocol. The results of these experiments are described in Table 2 (below).

**Table 2**

| **Sample** | **Wt.%** | **% Collagenase Inhibition** |
|---|---|---|
| Lactic Acid (L) | 1.25 | 40.32 |
| Pyruvic Acid (P) | 0.312 | 8.79 |
| L+P | 1.25 (L) + 0.312 (P) | 78.92 |

As demonstrated by the data described in Table 2 (above), a comparative composition provides synergistic inhibition of collagenase.

### Example 7 (comparative composition)

The anti-elastase activity of a comparative composition was compared to the anti-elastase activity of individual short chain fatty acids. Assays were performed using an elastase inhibitor screening kit from Sigma Aldrich (MAK 213 Kit); and following the manufacturer's protocol. The results of these experiments are described in Table 3 (below).

**Table 3**

| **Sample** | **Wt.%** | **% Elastase Inhibition** |
|---|---|---|
| Lactic Acid (L) | 0.625 | 44.89 |
| Pyruvic Acid (P) | 0.156 | 20.21 |
| L + P | 0.625 (L) + 0.156 (P) | 71.10 |

As demonstrated by the data described in Table 3 (above), a comparative composition provides synergistic inhibition of elastase.

### Example 8 (comparative composition)

The anti-tyrosinase activity of a comparative composition was compared to the anti-tyrosinase activity of individual short chain fatty acids. Assays were performed using a tyrosinase inhibitor screening kit from Sigma Aldrich (MAK 257 Kit); and following the manufacturer's protocol. The results of these experiments are described in Table 4 (below).

**Table 4**

| **Sample** | **Wt.%** | **% Tyrosinase Inhibition** |
|---|---|---|
| Lactic Acid (L) | 0.312 | 1.23 |
| Mandelic Acid (M) | 0.156 | 0.06 |
| L + M | 0.312 (L) + 0.156 (M) | 23.94 |

As demonstrated by the data described in Table 4 (above), a comparative composition provides synergistic inhibition of tyrosinase.

### Example 9 (comparative composition)

The anti-collagenase activity of a comparative composition was compared to the anti-collagenase activity of individual short chain fatty acids. Assays were performed using a collagenase inhibitor screening kit from Sigma Aldrich (MAK 293 Kit); and following the manufacturer's protocol. The results of these experiments are described in Table 5 (below).

**Table 5**

| **Sample** | **Wt.%** | **% Collagenase Inhibition** |
|---|---|---|
| Lactic Acid (L) | 1.25 | 12.71 |
| Mandelic Acid (P) | 0.625 | 8.57 |
| L + M | 1.25 (L) + 0.625 (M) | 43.16 |

As demonstrated by the data described in Table 5 (above), a comparative composition provides synergistic inhibition of collagenase.

### Example 10

Peels containing 16 wt.% lactic acid (L); 8 wt.% mandelic acid (M); and 4 wt.% of either pyruvic acid (P) or sodium pyruvate (P') [weight ratio of 4 : 2 : 1] were formulated, to compare the efficacy of pyruvic acid and sodium pyruvate, and to also evaluate the effect of pH on the activity of a peel.

Peels were diluted 1000X in buffer to prepare 0.1% working stock. The working stock was serially diluted 2X to test the peels at five doses (0.1 wt.%, 0.05 wt.%, 0.025 wt.%, 0.0125 wt.% and 0.00625 wt.%). The pH of all test samples and dilutions was measured before and after tyrosinase assay to exclude any effect of acid on the enzyme activity. The assay was performed using a commercial kit following manufacturer's protocol (e.g., MAK 257 Kit from Sigma Aldrich). LMP peels containing various concentrations of pyruvic acid at pH 2.5 ("R1 Peels"); LMP peels which were identically formulated to the R1 Peels, but with pH buffered to 3.6 ("R2 Peels"); and LMP' peels which were identically formulated to the R2 Peels, other than the replacement of pyruvic acid with sodium pyruvate ("R3 Peels"), were all evaluated. The data generated from these evaluations are described in Table 6 below.

**Table 6**

| **Peel** | **Wt.%** | **% Tyrosinase Inhibition** |
|---|---|---|
| **R1** | | |
| | 0.1 | 98.08221 |
| | 0.05 | 92.88508 |
| | 0.025 | 62.24947 |
| | 0.0125 | 26.08278 |
| | 0.00625 | 11.30976 |
| **R2** | | |
| | 0.1 | 99.56708 |
| | 0.05 | 99.89873 |
| | 0.025 | 98.54957 |
| | 0.0125 | 98.62214 |
| | 0.00625 | 85.9364 |
| **R3** | | |
| | 0.1 | 99.94533 |
| | 0.05 | 99.18071 |
| | 0.025 | 99.85245 |
| | 0.0125 | 97.90398 |
| | 0.00625 | 89.13448 |

As illustrated by the data described in Table 6 (above), the buffered peels containing pyruvic acid (R2 Peels) and sodium pyruvate (R3 Peels) demonstrated similar efficacy.

## Claims

1. A personal care composition comprising:
a complex comprising a plurality of fatty acids with less than six carbon atoms; and
a cosmetically acceptable carrier;
wherein the plurality of fatty acids comprises:
a first component comprising a first fatty acid or a salt thereof, wherein the first fatty acid comprises lactic acid or a salt thereof;
a second component comprising a second fatty acid or a salt thereof, wherein the second fatty acid comprises mandelic acid or a salt thereof; and
a third component comprising a third fatty acid or a salt thereof, wherein the third fatty acid comprises pyruvic acid or a salt thereof; and wherein the lactic acid, or a salt thereof, mandelic acid, or a salt thereof; and pyruvic acid, or a salt thereof are present in a weight ratio of about 4 : 2 : 1.

2. The personal care composition according to claim 1, wherein the cosmetically acceptable carrier comprises an ingredient selected from: a fragrance; a surfactant; a particle; a thickening agent; and a combination of two or more thereof, and/or wherein the composition is in a form selected from a body wash; a hand soap; a shower gel; a wipe; a lotion; a cream; an ointment; an antiperspirant; a peel; and a deodorant.

3. The personal care composition according to claim 1 or 2, comprising:
from 2 to 20 wt.%, optionally from 4 to 18 wt.%, further optionally 16 wt.% lactic acid, or a salt thereof;
from 2 to 12 wt.%, optionally from 4 to 10 wt.%, further optionally 8 wt.% mandelic acid, or a salt thereof; and
from 0.5 to 8 wt.%, optionally from 0.5 to 6 wt.%, further optionally 4 wt% pyruvic acid, or a salt thereof.

4. The personal care composition according to claim 3, comprising:
4 wt.% lactic acid, or a salt thereof;
2 wt.% mandelic acid, or a salt thereof; and
1 wt.% pyruvic acid, or a salt thereof.

5. The personal care composition according to any foregoing claim, wherein the salt of pyruvic acid is sodium pyruvate.

## Patentansprüche

1. Körperpflegezusammensetzung, umfassend:
einen Komplex, der eine Vielzahl von Fettsäuren mit weniger als sechs Kohlenstoffatomen umfasst; und
einen kosmetisch akzeptablen Träger;
wobei die Vielzahl von Fettsäuren umfasst:
eine erste Komponente, die eine erste Fettsäure oder ein Salz davon umfasst, wobei die erste Fettsäure Milchsäure oder ein Salz davon umfasst;
eine zweite Komponente, die eine zweite Fettsäure oder ein Salz davon umfasst, wobei die zweite Fettsäure Mandelsäure oder ein Salz davon umfasst; und
eine dritte Komponente, die eine dritte Fettsäure oder ein Salz davon umfasst, wobei die dritte Fettsäure Brenztraubensäure oder ein Salz davon umfasst; und
wobei die Milchsäure oder ein Salz davon, die Mandelsäure oder ein Salz davon und die Brenztraubensäure oder ein Salz davon in einem Gewichtsverhältnis von etwa 4 : 2 : 1 vorliegen.

2. Körperpflegezusammensetzung nach Anspruch 1, wobei der kosmetisch akzeptable Träger einen Inhaltsstoff umfasst, der ausgewählt ist aus: einem Duftstoff; einem Tensid; einem Partikel; einem Verdickungsmittel; und einer Kombination aus zwei oder mehreren davon, und/oder wobei die Zusammensetzung in einer Form vorliegt, die ausgewählt ist aus einem Körperwaschmittel; einer Handseife; einem Duschgel; einem Kosmetiktuch; einer Lotion; einer Creme; einer Salbe; einem Antitranspirant; einem Peeling; und einem Deodorant.

3. Körperpflegezusammensetzung nach Anspruch 1 oder 2, umfassend:
2 bis 20 Gew.-%, optional 4 bis 18 Gew.-%, weiter optional 16 Gew.-% Milchsäure oder ein Salz davon;
2 bis 12 Gew.-%, optional 4 bis 10 Gew.-%, weiter optional 8 Gew.-% Mandelsäure oder ein Salz davon; und
0,5 bis 8 Gew.-%, optional 0,5 bis 6 Gew.-%, weiter optional 4 Gew.-% Brenztraubensäure oder ein Salz davon.

4. Körperpflegezusammensetzung nach Anspruch 3, umfassend:
4 Gew.-% Milchsäure oder ein Salz davon;
2 Gew.-% Mandelsäure oder ein Salz davon; und
1 Gew.-% Brenztraubensäure oder ein Salz davon.

5. Körperpflegezusammensetzung nach einem der vorhergehenden Ansprüche, wobei das Salz der Brenztraubensäure Natriumpyruvat ist.

## Revendications

1. Composition de soins personnels comprenant :
un complexe comprenant une pluralité d'acides gras ayant moins de six atomes de carbone ; et
un support cosmétiquement acceptable ;
dans laquelle la pluralité d'acides gras comprend :
un premier composant comprenant un premier acide gras ou un sel de celui-ci, dans lequel le premier acide gras comprend de l'acide lactique ou un sel de celui-ci ;
un deuxième composant comprenant un deuxième acide gras ou un sel de celui-ci, dans lequel le deuxième acide gras comprend de l'acide mandélique ou un sel de celui-ci ; et
un troisième composant comprenant un troisième acide gras ou un sel de celui-ci, dans lequel le troisième acide gras comprend de l'acide pyruvique ou un sel de celui-ci ; et
dans laquelle l'acide lactique, ou un sel de celui-ci, l'acide mandélique, ou un sel de celui-ci ; et l'acide pyruvique, ou un sel de celui-ci sont présents selon un rapport pondéral d'environ 4:2:1.

2. Composition de soins personnels selon la revendication 1, dans laquelle le support cosmétiquement acceptable comprend un ingrédient choisi parmi : un parfum ; un tensioactif ; une particule ; un agent épaississant ; et une combinaison de deux ou plus de ceux-ci, et/ou dans laquelle la composition est sous une forme choisie parmi un nettoyant pour le corps ; un savon pour les mains ; un gel douche ; une lingette ; une lotion ; une crème ; une pommade ; un anti-transpirant ; un peeling ; et un déodorant.

3. Composition de soins personnels selon la revendication 1 ou 2, comprenant :
de 2 à 20 % en poids, éventuellement de 4 à 18 % en poids, en outre éventuellement 16 % en poids d'acide lactique, ou d'un sel de celui-ci ;
de 2 à 12 % en poids, éventuellement de 4 à 10 % en poids, en outre éventuellement 8 % en poids d'acide mandélique, ou d'un sel de celui-ci ; et
de 0,5 à 8 % en poids, éventuellement de 0,5 à 6 % en poids, en outre éventuellement 4 % en poids d'acide pyruvique, ou d'un sel de celui-ci.

4. Composition de soins personnels selon la revendication 3, comprenant :
4 % en poids d'acide lactique, ou d'un sel de celui-ci ;
2 % en poids d'acide mandélique, ou d'un sel de celui-ci ; et
1 % en poids d'acide pyruvique ou d'un sel de celui-ci.

5. Composition de soins personnels selon l'une quelconque des revendications précédentes, dans laquelle le sel d'acide pyruvique est le pyruvate de sodium.
